# EUROPEAN PATENT SPECIFICATION

(11) **EP 2 914 706 B1**
(45) Date of publication and mention of the grant of the patent: **18.03.2020**
(21) Application number: 13792185.4
(22) Date of filing: 04.11.2013
(51) Int. Cl.: C12N 1/06, C11B 1/10, C12M 1/00

(54) **METHOD AND APPARATUS FOR CELL LYSIS AND EXTRACTION**
VERFAHREN UND VORRICHTUNG FÜR ZELLLYSE UND -EXTRAKTION
PROCÉDÉ ET APPAREIL POUR LA LYSE ET L'EXTRACTION CELLULAIRES

(30) Priority: 05.11.2012 US 201213669085
(43) Date of publication of application: 09.09.2015
(73) Proprietor: Streamline Automation, LLC, Hunstville, AL 35805 (US)
(72) Inventor: CHEW, Geoffrey, Huntsville, AL 35802 (US); BOGGS, Tabitha, Huntsville, AL 35811 (US); DYKES, H., Waite, H., Jr., Huntsville, AL 35805 (US); DOHERTY, Stephen, J., Youngsville, NC 27596 (US)
(74) Representative: Invent Horizon IP
(86) International application number: PCT/US2013/068353
(87) International publication number: WO 2014/071324

(56) References cited:
- US-A1- 2009 234 146
- US-A1- 2011 130 551
- US-A1- 2011 192 793

## Description

### BACKGROUND OF THE INVENTION:

### Field of the Invention

The present invention relates to methods and systems for lysing and processing cells to extract lipids, proteins, carbohydrates, metabolites, and/or other cellular components. In particular, the invention is a method and a system for extracting lipids and other cellular components from cells in which a cell lysate comprising an ionic liquid is used to lyse cells.

### Description of Related Art

### Ionic liquids

US 2009/0234146 A1 discloses a method for the direct transesterification and extraction of biolipids from biomass, including plants, yeast, and algae. The method requires a cosolvent system containing an ionic liquid solvent and polar covalent solvent to lyse cells and for the formation of separate hydrophilic and hydrophobic phases.

US 8,450,111 B2 discloses a one-step process for the lysis of microalgae cells by 1-butyl-3-methylimidazolium chloride (BMIM Cl) and the separation of cellular lipids for use in biofuel production. US 8,211,307 discloses an algae cell lysis method using a hydrophilic ionic liquid to lyse algae cells and then form hydrophobic and hydrophilic phases that are separated. After separation of the hydrophilic and hydrophobic phases, the hydrophilic ionic liquid may be recovered for reuse by adding a salt to the isolated hydrophilic phase to form separate aqueous salt and ionic liquid phases. US 8,303,818 B2 discloses a method for extracting material from algae cells in which algae cells are contacted with a pure ionic liquid or an active ionic liquid comprising at least 65% ionic liquid. US 8,211,307 B2 discloses a method for processing algae cells by mixing wet algae cells with a hydrophilic ionic liquid to form a cell lysate and salting out the cell lysate to form separate hydrophobic, ionic liquid, and aqueous salt solution phases.

The aforementioned processes are capable of lysing and processing algae cells using an ionic liquid and recycling the ionic liquid after it is used to lyse cells. Ionic liquids, having lower vapor pressures than volatile organic solvents used for chemical cell lysis, are generally safer to transport and use. Ionic liquids, however, are much more expensive than organic solvents and must therefore be recycled with minimal losses. The process of recycling ionic liquids consumes time and energy and may involve heating the ionic liquid to remove water. Repeatedly heating ionic liquids during recycling may gradually cause some ionic liquids to decompose and/or limit their capacity to lyse cells. Salting out of ionic liquids consumes time and materials and phosphate salts that are effective for salting-out ionic liquids can be damaging to the environment. Accordingly, there is a need for further improved methods and systems for processing algae, and other biomass, that reduce the time, energy, temperature, and materials required for purifying the ionic liquid before reuse.

The formation of separate hydrophobic and hydrophilic phases may be difficult when the volumes of lipid extracted are relatively small compared to the total volume of cell lysate. Accordingly, there is a need for methods and systems for extracting lipid from algae, and other biomass, that improve phase separation.

Accordingly, embodiments of the present invention preferably seek to mitigate, alleviate or eliminate one or more deficiencies, disadvantages or issues in the art, such as the above-identified, singly or in any combination, by providing a system, and a method for processing algae and other biomass, according to the appended patent claims.

### BRIEF SUMMARY OF THE INVENTION:

The present invention provides a method, and a system for extracting lipids, carbohydrates, proteins, metabolites, small molecules, and/or other components of micro- and macro-algae. These cellular components may be, or be used to produce, biofuels, nutrients, pharmaceuticals, chemicals, and/or other useful products.

The invention also provides methods and systems for lysing and/or extracting prokaryotic or eukaryotic cells and methods and systems for extracting lipids from cells.

The invention is based, in part, on the discovery that a cell lysate formed by mixing an ionic liquid (IL) with algae can be used to lyse additional algae after solid debris is removed from the algae lysate to form a clarified lysate. This allows for multiple rounds of cell lysis without intervening IL recovery or purification steps. The process involves mixing an IL-containing clarified algae cell lysate with algae cells to form a crude algae cell lysate and removing solid debris from the crude lysate to form a clarified algae cell lysate.

In some embodiments, the process is used for lipid extraction wherein the lipid content of the lysate increases with each round of lysis and, after a number of iterations of this process, a clarified cell lysate is allowed or caused to form separate lipid and aqueous phases. The lipid phase is isolated and optionally processed to remove residual IL. A fatty acid may be mixed with a clarified lysate to improve phase separation and lipid recovery. Water, methanol, or a water-methanol mixture may be added to a crude or a clarified cell lysate to enhance precipitation of solid debris before removal of solid debris to form a clarified cell lysate. The aqueous phase may be processed to produce purified IL to be reused to begin a subsequent iterative cell lysis process.

### BRIEF DESCRIPTION OF THE DRAWINGS:

These and other aspects, features and advantages of which embodiments of the invention are capable of will be apparent and elucidated from the following description of embodiments of the present invention, reference being made to the accompanying drawings, in which
FIG. 1 is a flow diagram of a method according to the invention,
FIG. 2 is a schematic of a general system according to the invention, and
FIG. 3 is a schematic of a specific embodiment of a system according to the invention.

### DETAILED DESCRIPTION OF THE INVENTION:

Specific embodiments of the invention are described with reference to the accompanying drawings. This invention may, however, be embodied in many different forms and should not be construed as limited to the embodiments set forth herein. Rather, these embodiments are provided so that this disclosure will be thorough and complete, and will fully convey the scope of the invention to those skilled in the art. In the drawings, like numbers refer to like elements.

The following description focuses on an embodiment of the present invention applicable to the extraction of lipids from algae and in particular to the extraction of triacylglycerols and free fatty acids for the production of biodiesel. However, it will be appreciated that the invention is not limited to this application but may be applied to the extraction many other components of algae cells and even to the extraction of products from non-algae cells that can be lysed using the disclosed method and system.

One aspect of the invention involves a method in which an IL-containing clarified cell lysate is mixed with algae to lyse algae cells and form a crude IL-containing cell lysate.

Another aspect of the invention involves a method in which sequential batches of algae are mixed with clarified IL-containing cell lysates formed from crude IL-containing lysates of a preceding batch of algae.

Another aspect of the invention involves a method in which a fatty acid is mixed with a clarified IL-containing cell lysate before the partition of an IL-containing hydrophilic phase from a lipid-containing hydrophobic phase.

Another aspect of the invention is a system for processing algae cells comprising a lysis reactor in fluid communication with a solids removal chamber, a liquids separation vessel in fluid communication with the solids removal chamber, and a distillation unit in fluid communication with the liquids separation vessel.

The term "lysis" as used herein with respect to algae cells, involves the dissolution of the cell wall as well as the cell membrane.

An ionic liquid (IL), as used herein, refers to an IL that is a liquid below 150°C. A low-melting IL is an IL with a melting point below about 100°C. 1-ethyl-3-methylimidazolium chloride [EMIM]Cl and [BMIM]Cl are examples of ionic liquids having melting temperatures of between 50°C and 100°C. A preferred IL is a hydrophilic IL that is a liquid at a temperature below 50°C. 1-ethyl-3-methylimidazolium acetate ([EMIM]Ac), 1-methyl-3-octylimidazolium chloride ([OMIM]Cl), 1-Hexyl-3-methylimidazolium chloride ([HMIM]Cl), 1-Hexyl-3-methylimidazolium iodide ([HMIM]I), and 1-ethyl-3-methylimidazolium triflate ([EMIM]OTf) are examples of preferred ILs. [EMIM]Ac is most preferred. A pure IL refers to a liquid composed entirely of ions. Molten sodium chloride, for example, is not considered as an IL because the melting point of sodium chloride is 801 °C., which is above the herein defined melting point of an IL of less than about 150°C. Some ILs are hygroscopic, which results in the absorption of moisture from the air. Consequently, an IL may contain a small percentage of water. Herein, a pure IL contains less than one-tenth of a percent water by mass.

An aqueous IL solution contains water and an IL. An active IL contains less than 45% water by mass and preferably less than 40% water by mass, and most preferably less than 35% water by mass. An inactive IL solution contains 55% or more, preferably 60% or more, and most preferably 65% or more water by mass. If an insoluble component is present, the mass percentages of IL and water refer to the percentages of the dissolved components only. For example, the mass of intact cells added to an IL solution and/or precipitated cell debris resulting from lysis are not included in the mass of the IL solution.

An IL may consist of a single species of cation and a single species of anion (a single IL) or an IL may contain 2 or more cations and/or 2 or more anions (composite ILs). For example, 1-Ethyl-3-methylimidazolium acetate ([EMIM]Ac) is a hygroscopic, low-melting point IL. A mixture of 1-Buttyl-3-methylimidazolium Chloride ([BMIM]Cl) and [EMIM]Ac is also a low-melting point composite IL. A mixture of [OMIM]Cl and [HMIM]Cl is also a low-melting point composite IL.

As used herein, "algae" and "algae cells" refers to fresh water and marine algae, microalgae, and macroalgae. The terms "algae" and "algae cells" may be used interchangeably to indicate algae cells present in a suspension of single celled algae species or cells present in macroalgae. Macroalgae may be chopped, shredded, or whole when lysed. Examples of algae include species of *Amphiprora, Bacillariophyceae, Botryococcus, Chlamydomonas, Chlorella, Chlorococcum, Chlorophyceae, Chrysophyceae, Cylindrotheca, Dunaliella, Laminaria, Nannochloris, Navicula, Neochloris, Phaeodactylum, Pleurochrysis, Prymnesiophyceae, Sargassum, Scenedesmus, Selenastrum, Tetraselmis sp, and Thalassiosira.* Examples of species of algae include *Botryococcus braunii, Chlamydomonas reinhardtii, Chlamydomonas moewusii, Chlorella vulgaris, Chlorella pyrenoidosa, Chlorella ellipsoidea, Chlorella vulgaris, Chlorella protothecoides, Dunaliella tertiolecta, Laminaria digitata, Nannochloropsis oculata, Nannochloropsis salina, Neochloris oleoabundans, Phaeodactylum tricornutum, Pleurochrysis carterae, Sargassum muticum, Scenedesmus dimorphus, Selenastrum capricornotum, and Thalassiosira pseudonana.* The list of algae provided is provided for illustrative purposes and is not intended to be limiting. The algae being lysed may be from a single strain of algae or a mixture of strains or species, including mixtures of microalgae and macroalgae, for example.

As used herein, a clarified cell lysate is a crude cell lysate that has been clarified by removing solid debris, for example by filtration, centrifugation, and/or removal of solids that settle by gravitation. In some cases, a precipitating agent may be used to enhance precipitation. In some cases filtration may be enhanced by coatings, surface treatments, or the use of materials that use molecular polarity to speed filtration relative to an unenhanced filter material.

As used herein, the singular forms "a", "an" and "the" are intended to include the plural forms as well, unless expressly stated otherwise. It will be further understood that the terms "includes," "comprises," "including" and/or "comprising," when used in this specification, specify the presence of stated features, integers, steps, operations, elements, and/or components, but do not preclude the presence or addition of one or more other features, integers, steps, operations, elements, components, and/or groups thereof. It will be understood that when an element is referred to as being "connected" or "coupled" to another element, it can be directly connected or coupled to the other element or intervening elements may be present.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

### Method

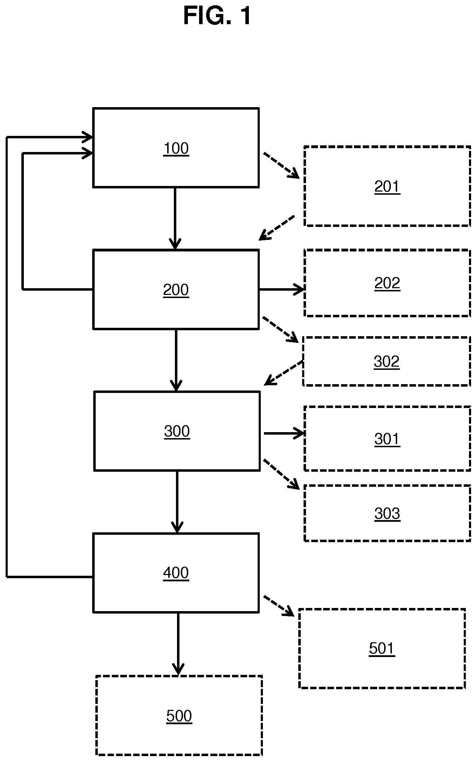

A flow diagram representing the basic method is shown in FIG. 1 in which solid lines represent required steps and dashed lines represent optional steps. Intact algae cells, optionally pre-treated by washing or soaking in a hypertonic solution, are mixed with pure IL, dewatered recycled IL from step 400, and/or IL-containing clarified algal cell lysate in step 100. The IL-containing clarified algae lysate may be formed by mixing a substantially pure IL with algae, as described in US 8,211,307, US 2011/0076748, or US 8,388,846 followed by solid debris removal in step 200. The hydrophobic IL may be a single IL or a mixture of ILs. The clarified lysate comprises IL in an amount of at least 65% by weight, preferably 75% by weight, more preferably 85% or 90% by weight. The algae may contain between 5% and 90% extracellular water by weight and is mixed with IL-containing clarified lysate in a ratio of from about 1:5 to 1:20 and more preferably from about 1:10 to about 1:20 by volume. The total amount of water in the mixture of algae and clarified lysate preferably does not exceed about 35% by weight. Mixing is preferably performed at a temperature of between 20°C and 50°C but may performed at higher or lower temperatures depending, for example, on the melting point and lysing activity of the IL. A higher temperature may be advantageous, for example, when using an IL having a melting temperature above 50°C. A lower temperature may be used, for example, when the ambient temperature and melting point of the ionic liquid are below 20°C and adequate lysis is achieved at lower temperatures. Mixing takes place for a time sufficient to lyse substantially all intact algae or a target percentage of intact algae. The target may be 99, 98, 95, 90, 85, or 80 percent lysis, for example. Mixing may be achieved by any suitable mixing means including impeller(s), stirring bar(s), paddle mixer(s), and pulse air mixer(s). The minimum duration of mixing depends on the algae, IL, water content, IL content, temperature, and efficiency of mixing and may range, for example, from 1 to 60 minutes.

Optimal conditions for lysis, including weight % of IL, composition of IL, maximum water content of lysate, mixing time, mixing temperature, and mixing mechanism may be determined for different algae cell types or non-algae cell types to be lysed and/or extracted. As an example, the IL may be [EMIM]OAc, the temperature may be 20°C-30°C, the mixing time may be from 5 min to 30 min, and the weight ratio of clarified lysate to algae may be from 5:1 to 20:1.

The crude lysate resulting from the mixed algae and IL-containing clarified lysate normally contains solid cell debris that is removed in step 200 to generate a clarified lysate that may be used to lyse additional algae in step 100. The solid debris may be removed by a suitable means such as filtration, centrifugation, or settling and scraping. The crude lysate may be passed through a first filter, or pre-filter, to remove solid debris and additionally or alternatively passed through a second filter having a finer mesh than the first filter. Examples of pre-filters include 20, 30, 40, and 80 multilayer mesh filters. Examples of second filters include 2 micrometer, 6 micrometer, 10 micrometer, 30 micrometer and 50 micrometer single layer filters of the type used for water purification systems. Additional filtration steps with progressively smaller filter pore sizes may be performed if needed. The filter medium may be enhanced with a surface treatment that uses molecular polarity to increase the speed with which one or more liquids is transported through the filter media compared to simple gravity driven flow. This transport may be further enhanced through the use of differential pressure across the filter medium.

The solid debris may be washed with water in order to remove residual IL and recoverable lipids from the debris. The IL and lipids may then be separated from the wash water. Washing may be repeated if necessary.

In some cases, a precipitating agent may be mixed with the crude lysate in step 201 to enhance and/or accelerate the formation of solid debris before the solid debris is removed. The precipitating agent may be, for example, an acid or base used to change the pH of the crude lysate. The pH may be selected, for example, to precipitate a product that is to be isolated from the solid debris or to ensure that a desired product remains soluble in the lysate. Methanol or a methanol-water mixture may additionally or alternatively be used as a precipitating agent in a weight ratio from about 0.5:1 to about 2:1, and preferably about 1:1 methanol-water to crude lysate. If methanol or methanol/water is not used to enhance precipitation, the clarified lysate formed after removal of solid debris in step 200 may be distilled or dried in step 400 without passing through separation step 300 for reuse in lysis step 100. Acetone and or ethyl acetate may be used in addition to or as an alternative to methanol or methanol-water as a precipitating agent. Accordingly, once a methanol-water solution is added to a crude lysate, the resulting solution is not a part of the clarified lysate that can be used for further cell lysis. Additionally or alternatively, a change in temperature may be used to enhance precipitation. The precipitate, once removed from the crude lysate, may be further processed in step(s) 202 to isolate one or more desired metabolites, macromolecules, or other desired product(s). Methanol or methanol-water may, as an alternative to being used as a precipitating agent, be added to a clarified lysate to enhance phase separation between hydrophilic and hydrophobic phases.

In some cases, for example when extracting lipids from algae, a free fatty acid may be mixed with the clarified lysate in step 302 to enhance phase formation and separation in separating step 300. For example, oleic acid may be mixed into the clarified lysate before or during the transfer of the clarified lysate to a liquid separation vessel where the clarified lysate rests to allow the formation of separate hydrophobic and hydrophilic phases in separation step 300. Other fatty acids may alternatively or additionally be used to enhance phase formation and separation. The amount of fatty acid mixed with clarified lysate may be between 0.5% and 1.5% by weight and preferably around 0.7% by weight of the total clarified cell lysate.

The separation step 300 may use membranes that selectively allow chemical molecules to pass through or preferentially prevent molecules from passing through. Molecular properties such as polarity may be used as the basis for this separation. The membrane separation may be enhanced through the use of differential pressure across the membrane, or may be used in conjunction with gravity driven separation.

Small amounts of IL may be found in the lipid phase. This IL can be recovered, for example, by adding a nonpolar solvent to the isolated lipid phase to separate the IL from the lipid phase. The IL may then be recycled to step 100, for example. In some cases a precipitate may form in addition to the hydrophilic and hydrophobic phases. The precipitate may form at the interface of the hydrophilic and hydrophobic phases and/or at the bottom of the vessel. In such cases the precipitate(s) is (are) removed and optionally processed in step 303.

The clarified lysate may be mixed with algae to produce a new crude lysate in step 100 or the clarified lysate may be allowed or caused to form separate hydrophobic and hydrophilic phases in step 300. The hydrophobic (lipid) phase comprises constituents of the algae that are not substantially soluble in the IL-containing aqueous phase. Constituents present in the hydrophobic phase may include triacylglycerols (TAGs), which contain fatty acids that may include, for example, eicosenioc acid, eicosedienioc acid, arachadonic acid, hexadecadienoic acid, hexadecatrienoic acid, palmitic acid, palmitoleic acid, stearic acid, oleic acid, linoleic acid, and linolenic acid. The aqueous phase comprises water, IL, and water soluble constituents of the lysed algae. The phases may be separated using any suitable separation means, such as sequentially removing the hydrophilic phase and the hydrophobic phase from the bottom of the vessel or removing the top phase from the top of the vessel. After the hydrophobic phase has been isolated, it may be further processed in step(s) 301 from its bio-crude state using separation and/or chemical transformation processes. For example, TAGs may be isolated and converted into fatty acid methyl esters (FAMEs) for use as biodiesel.

The hydrophilic phase comprising water and IL may be distilled in step 400 to separate IL from water. The IL-containing hydrophilic phase is heated to a temperature of above 100°C and preferably to a temperature of between 110°C and 120°C to remove water, which may be condensed in step 500 for recycling or release from the system. If methanol or water/methanol was used as a precipitating agent, the methanol may be recovered together with the water in step 500 or the methanol may be collected separately in step 501. The hydrophilic phase is preferably sparged with heated air or a heated inert gas during distillation to disrupt surface tension and increase the liquid surface area to accelerate the distillation process, reduce the distillation temperature, reduce the time during which the IL is exposed to elevated temperature, and/or to accelerate water removal. Reverse osmosis may alternatively or additionally be used for removing water and/or methanol from the hydrophilic phase in step 400 so that the IL may be reused for lysis.

The IL recycling step 400 may use membranes that selectively allow chemical molecules to pass through or preferentially prevent molecules from passing through. Molecular properties such as polarity may be used as the basis for this separation. The membrane separation may be enhanced through the use of differential pressure across the membrane, or may be used in conjunction with gravity driven separation.

The cycling of IL-containing clarified lysate through steps 100 and 200 may be repeated a limited number of times. For example, steps 100 and 200 may be cycled 2 times, 5 times, 10 times, 20 times 30 times, 40 times, or 50 times. Monitoring of the water content of the clarified lysate in 200 and/or the crude lysate resulting from step 100 may be used to control whether a clarified lysate is recycled to lysis step 100 or moved on to separation step 300. The clarified lysate, however, contains a higher proportion of water with each cycle. At some point, the combined water in the algae being lysed and the water in the clarified lysate will reach 45%, or preferably 40%, or more preferably 35%, which may result in diminished algae lysis. Therefore, the clarified lysate may be passed onto separation step 300 before the threshold of 45% combined total water in the lysing mixture is reached. Accordingly, algae cell suspension or macroalgae comprising less water allow the clarified IL-containing lysate to be used for more cycles of lysis than algae comprising more water. The number of cycles though steps 100 and 200 may also be limited by the efficiency of extraction. The optimum number of cycles before moving to liquid separation step 300 may be determined experimentally by comparing the amount of desired product extracted after each cycle. For example, if the desired product is TAG, steps 100 and 200 may be repeated for a number of cycles with a small aliquot taken after each cycle. Each sample may be analyzed to determine the amount of desired product in the sample. The amount of desired product should increase with each cycle in proportion with the amount of algae being lysed. The number of cycles at which the increase in amount of desired product does not increase in relative proportion to the amount of algae lysed minus 1, for example, may be selected as the number of times to cycle between steps 100 and 200. Recovering the IL by distilling away water does not appear to diminish the ability of the IL to lyse algae. For example, [EMIM]Ac recycled through 50 rounds of dewatering by distillation maintains lysing activity. Water may alternatively be removed from the IL-containing hydrophilic phase by heating with passive or active solar heat, microwaves, or heat from combustion without recovery of water.

The above method has been demonstrated using [EMIM]Ac or [EMIM]OTf with *Tetraselmis sp., Chlorella pyrenoidosa, Chlorella ellipsoidea, Chlorella vulgaris, Laminaria digitata, Nannochloropsis oculata, Sargassum muticum,* and *Scenedesmus dimorphus.*

### System

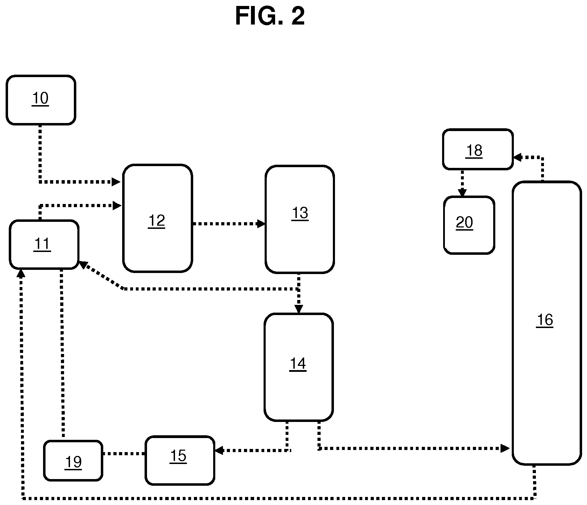

A generalized system for performing the method of the invention is shown in FIG. 2. A source of algae 10 and a source of IL-containing clarified algal cell lysate 11 are in communication with a lysis reactor unit 12. The lysis reactor unit 12 comprises means for mixing the algae and clarified lysate and is in fluid communication with a solids removal unit 13. The algae may be, for example, an algae paste or algae suspension comprising as much as 85% water and algae cells comprising from 25% to 60% oil. Crude lysate formed in the lysis reactor unit 12 are transferred to the solids removal unit 13 where solid debris is removed from the crude lysate to form a clarified lysate. Solids removal unit 13 may comprise, for example, a prefilter having a first mesh size and one or more filters having a smaller mesh than the prefilter. Solids removal unit 13 may alternatively or additionally comprise a centrifuge or any other suitable means for removing solid debris from a cell lysate. Clarified lysate may be transferred back to the lysis reactor unit 12 to be mixed with algae cells to produce a new crude cell lysate or the clarified cell lysate may be transferred to liquid separation unit 14 where lipid containing hydrophobic and IL-containing hydrophilic phases separate from one another. The lipid containing hydrophobic phase is transferred for removal from the system or optionally for further processing to lipid collection port 15. Residual IL may optionally be recovered from the lipid by IL recovery unit 19. The IL-containing hydrophilic phase is transferred from the liquid separation unit 14 to water removal unit 16. Water removal unit preferably comprises a distillation unit where the IL-containing hydrophilic phase is heated, for example, to between 110°C and 120°C to remove water. Water may be recovered by a condenser unit 18 and removed from the system or recycled via water container 20. Water removal unit 16 may additionally or alternatively comprise a reverse osmosis unit for removing water and optionally precipitating agent other unwanted solutes.

### Example

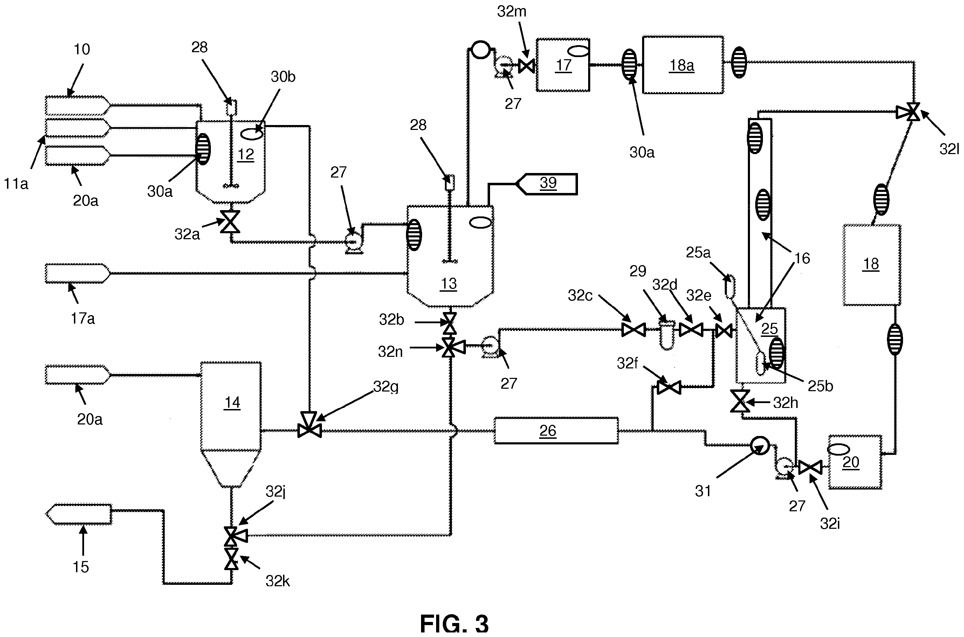

FIG. 3 shows an embodiment of a system for performing an algae processing method according to the invention. A lysis reactor 12 is configured to receive algae from a source of algae 10 and to receive an IL-containing clarified algae cell lysate from a solids separation unit 13 via valves 32b and 32n, pump 27, valve 32c, filter 29, valves 32d and 32f, optionally in-line mixer 26, and valve 32g. At start-up, no algae has been lysed and there is no IL-containing clarified lysate so lysis reactor 12 is configured to receive a substantially pure IL from a source of IL 11a and to receive water from water source 20a. Lysis reactor 12 is equipped with a means for mixing algae with IL or IL-containing clarified algae lysate. The mixing means may be an impeller as shown or any other suitable mixing means. Lysis reactor 12 is also provided with a temperature sensor 30a, a level sensor 30b, and a water sensor 30c.

A solids separation unit 13, in this case a prefilter, is configured to receive crude cell lysate from lysis reactor unit 12 via valve 32a and pump 27 and to remove solid debris from crude cell lysate to produce a clarified algae cell lysate. A stirrer 28 circulates liquid in a chamber of the separation unit 13 to direct solids toward the sides of the chamber to minimize clogging of a mesh filter (not shown) at the bottom of the chamber. The solids separation unit 13 is configured to deliver a clarified algae cell lysate to either lysis reactor unit 12 or separation unit 14 via valves 32b and 32n, pump 27, valve 32c, filter 29, valves 32d and 32f, optionally in-line mixer 26, and valve 32g. Separation unit 14 comprises a vessel configured to receive clarified algae cell lysate from solid separation chamber 13, for the formation of separate lipid-containing hydrophobic and IL-containing hydrophilic phases, and preferably for the isolation of the hydrophilic and hydrophobic phases from one another. The separation unit 14 in this embodiment is configured similarly to a separatory funnel such that a lower hydrophilic phase may be removed from the bottom of the vessel and transferred to distillation unit 16 via valves 32j and 32n, pump 27, valve 32c, filter 29, and valves 32d and 32e. After the IL-containing hydrophilic phase is removed from liquid separation vessel 14, the lipid-containing hydrophobic phase is removed through valves 32j and 32k and transferred to lipid collection port 15, which may be connected, for example, to a storage container or pipeline (not shown).

The solids separation unit 13 may comprise multiple filtration chambers arranged in parallel to allow for continuous batch production by, for example, rotating between filter chambers and filter scrapers/cleaners. IL contaminating the solid debris may be recovered, for example, by water extraction of the solid debris.

The embodiment shown in FIG. 3 comprises an optional reservoir 17 containing an optional precipitating agent, in this case methanol. A precipitating agent may be used, for example, after a number of lysis cycles and before transfer of clarified cell lysate to the liquid separation unit 14. In this example, methanol is pumped out of reservoir 17 through valve 32m by pump 27, through a flow meter 30, and into solids separation unit 13. Stirrer 28 is used to mix the precipitating agent with the crude lysate. The methanol precipitating agent separates with the hydrophilic phase in liquid separation unit 14 and is transferred to the water removal unit 16 unit in the hydrophilic phase.

The water removal unit 16 in this embodiment is a distillation unit and comprises a sparger 25 that introduces compressed air from a compressed air source 25a to a sparging outlet 25b in the sparger. The air may heated to approximately the temperature of the liquid through which it passes. Temperature sensors 30a monitor the temperature at various locations in the distillation unit, including the temperature of the liquid being heated and the gas generated. Methanol, having a lower boiling point than water comes off the distillation apparatus first and is directed through valve 32l to condenser 18a which, in turn, flows into reservoir 17. Temperature sensors 30a monitor temperatures before and after the condenser. Water (steam) comes off the distillation apparatus after methanol and is directed through valve 32l to condenser 18 which, in turn, flows into water reservoir 20. Water may be returned to the lysis unit via valve 32i through a pump 27, a flow meter 31, optionally through in-line mixer 26, and valve 32g. Supplemental water and methanol may be provided from supplemental water supplies 20a and supplemental methanol supply 17a.

A source of fatty acid 39, in this case oleic acid, is configured to deliver oleic acid into the solids separation unit 13. Fatty acid is introduced after multiple rounds of lysis and, for example, immediately before the clarified lysate is transferred to liquid separation unit 14 via valves 32b and 32n, a pump 27, valve 32c, filter 29, valve 32f, in-line mixer 26, and valve 32g. In-line mixer 26 is used to ensure that the fatty acid is completely mixed with the clarified cell lysate before the clarified lysate reached the liquid separation unit 14. Valves 32c and 3d facilitate changing filter 29. Flow meters 31, pumps 27, pressure sensors, temperature sensors 30a, and level sensors 30b may be located at any number of positions within the system in addition to or as an alternative to the positions shown in FIG. 3. The fatty acid may alternatively be added to and mixed with the clarified cell lysate at any point between the solids removal unit 13 and the liquid separation unit 14.

The system, in this embodiment, produces product streams including cell debris from the solids separation unit 16, purified (dried or dewatered) IL, water and methanol from dewatering unit 16, and biocrude from separations unit 14.

The present invention is described using lipid extraction from algae cells as an example. One will appreciate that the present invention can also be used to extract a component found in the lipid phase, solid precipitate (solid debris), or the hydrophilic phase of the clarified cell lysate. Thus, the method and system may be used to process algae for the purpose of extracting other materials from algae and other cell types, including polysaccharides, sugars, xanthophylls, astaxanthin and other carotenoids, nucleic acids, proteins, and omega-fatty acids. Lipid soluble products may be isolated from the lipid phase recovered from the liquids separation vessel using techniques known in the art. Insoluble products or products made insoluble by a precipitating agent in the solids removal chamber may be isolated from the precipitated solid using techniques known in the art. Products found in the hydrophilic phase of the clarified cell lysate may be extracted using extraction methods known in the art.

Embodiments of the present invention are described herein with reference to a flowchart diagram. It will be understood that some or all of the illustrated blocks may be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. Valves, sensors, pumps, meters, the distillation unit, chambers, reactor, vessels, and heating may all be controlled by microprocessor or computer controller. Computer readable code may be stored on a tangible computer readable medium and used to control the operation of the system and performance of the method. Any suitable computer readable medium may be utilized including hard disks, CD-ROMs, optical storage devices, a transmission media such as those supporting the Internet or an intranet, or magnetic storage devices.

The invention has been described with reference to a limited number of preferred embodiments. One of skill in the art will readily appreciate that the number of described embodiments is limited for the sake of brevity and clarity and that the invention is not limited to the embodiments described. Many other embodiments may be substituted for those described herein without departing from the scope of the invention.

## Claims

1. A method for processing cells, said method comprising:
a) mixing (100) cells with a first ionic liquid (IL) containing clarified cell lysate to form a crude cell lysate;
b) removing debris (200) from said crude cell lysate to form a second clarified cell lysate;
c) separating lipid-containing hydrophobic and ionic liquid-containing hydrophilic phases (300) present in the second clarified cell lysate; and
d) isolating the lipid-containing hydrophobic phase from the ionic liquid-containing hydrophilic phase;
wherein the first clarified cell lysate comprises at least 65% hydrophilic ionic liquid by weight and the total amount of water in the crude cell lysate formed in step a) is not more than 45% by weight.

2. The method of claim 1, wherein the cells are algae cells.

3. The method of claim 2, and further comprising mixing (302) a fatty acid with the second clarified cell lysate before forming separate lipid-containing hydrophobic and ionic liquid-containing hydrophilic phases.

4. The method of claim 3, wherein said fatty acid is added in an amount of about 0.5% to about 1.5% of the second clarified cell lysate by weight.

5. The method of claims 2 to 4, wherein said algae cells and first clarified cell lysate are mixed in a weight ratio of between 1:5 and 1:20.

6. The method of any of claims 1-5, wherein said mixing of cells and first clarified cell lysate is performed at a temperature of between 20 °C and 50 °C for a duration of between 1 and 60 minutes.

7. The method of any preceding claim, and further comprising mixing (201) the crude cell lysate with a precipitating agent before said removing debris.

8. The method of claim 7, wherein said precipitating agent is selected from the group consisting of an acid, a base, methanol, 50% methanol in water, and combinations thereof.

9. The method of any of claims 1-8, wherein the cells are algae cells in the form of an algae cell suspension comprising between 10% and 90% water by weight.

10. The method of any of claims 2-9, wherein the algae cells are in the form of whole, shredded, or chopped macroalgae.

11. The method of any preceding claim, wherein the ionic liquid is selected from the group consisting of 1-ethyl-3-methylimidazolium chloride, 1-butyl-3-methylimidazolium chloride, 1-ethyl-3-methylimidazolium acetate, 1-methyl-3-octylimidazolium chloride, 1-Hexyl-3-methylimidazolium chloride, 1-Hexyl-3-methylimidazolium iodide, 1-ethyl-3-methylimidazolium triflate, and combinations thereof.

12. The method of any preceding claim, and further comprising repeating the cycle of steps a) and b) between 2 and 50 times before proceeding to step c).

13. The method of any preceding claim, and further comprising removing water from the ionic liquid-containing hydrophilic phase of said second clarified cell lysate.

14. A system for processing cells, said system comprising:
a source of hydrophilic ionic liquid (11, 11a),
a temperature controlled lysis reactor unit (12) comprising means for mixing (28);
a temperature controlled solids removal unit (13) comprising a stirrer (28);
a liquid separation unit (14); and
a water removal unit (16)
wherein:
said lysis reactor unit (12) is in fluid communication with said solids removal unit (13) such that a crude cell lysate may be transferred from the lysis reactor unit (12) to the solids removal unit (13) and an ionic liquid (IL) containing clarified cell lysate may be transferred from the solids removal unit (13) to the lysis reactor unit (12);
said lysis reactor unit (12) is in communication with a source of cells (10) and is configured for mixing said cells and said IL-containing clarified cell lysate;
said solids removal unit (13) is in fluid communication with said liquid separation unit (14) in such a way that clarified cell lysate may be transferred either to the liquid separation unit (14) or to the lysis reactor unit (12);
said liquid separation unit (14) is in fluid communication with said water removal unit (16) and a fluid reservoir such that fluid may be transferred from the liquid separation unit (14) to the water removal unit (16) or to said fluid reservoir;
said water removal unit (16) is in fluid communication with said lysis reactor unit (12) such that ionic liquid in the water removal unit (16) may be transferred to the lysis reactor (12); and
the system is configured to transfer a clarified cell lysate to said liquids separation unit (14) dependent upon a monitored water content of clarified lysate in said solids removal unit (13).

15. The system of claim 14, wherein said water removal unit (16) comprises a distillation unit comprising a sparger (25) configured for sparging a liquid in the distillation unit with a heated gas.

16. The system of claim 14, wherein said distillation unit is in fluid communication with said solids removal unit (13) and is configured such that vapor from the distillation unit is condensed to a liquid and transferred to the solids removal unit (13).

## Patentansprüche

1. Verfahren zur Verarbeitung von Zellen, wobei das Verfahren umfasst:
a) Mischen (100) von Zellen mit einer ersten ionischen Flüssigkeit (IL), die geklärtes Zelllysat enthält, um ein rohes Zelllysat zu bilden;
b) Entfernen von Trümmern (200) aus dem rohen Zelllysat, um ein zweites geklärtes Zelllysat zu bilden;
c) Trennen einer lipidhaltigen hydrophoben Phase und einer ionische Flüssigkeit enthaltenden hydrophilen Phase (300), die in dem zweiten geklärten Zelllysat vorhanden sind; und
d) Isolieren der lipidhaltigen hydrophoben Phase von der ionische Flüssigkeit enthaltenden hydrophilen Phase; wobei das erste geklärte Zelllysat mindestens 65 Gew.-% hydrophile ionische Flüssigkeit umfasst und die Gesamtmenge an Wasser in dem in Schritt a) gebildeten rohen Zelllysat nicht mehr als 45 Gew.-% beträgt.

2. Verfahren nach Anspruch 1, wobei die Zellen Algenzellen sind.

3. Verfahren nach Anspruch 2, und ferner umfassend das Mischen (302) einer Fettsäure mit dem zweiten geklärten Zelllysat, bevor getrennte lipidhaltige hydrophobe und ionische Flüssigkeit enthaltende hydrophile Phasen gebildet werden.

4. Verfahren nach Anspruch 3, wobei die Fettsäure in einer Menge von etwa 0,5 bis etwa 1,5 Gew.-% des zweiten geklärten Zelllysats zugegeben wird.

5. Verfahren nach den Ansprüchen 2 bis 4, wobei die Algenzellen und das erste geklärte Zelllysat in einem Gewichtsverhältnis zwischen 1:5 und 1:20 gemischt werden.

6. Verfahren nach einem der Ansprüche 1-5, wobei das Mischen von Zellen und des ersten geklärten Zelllysats bei einer Temperatur zwischen 20 °C und 50 °C für eine Dauer zwischen 1 und 60 Minuten durchgeführt wird.

7. Verfahren nach einem der vorhergehenden Ansprüche, und ferner umfassend das Mischen (201) des rohen Zelllysats mit einem Fällungsmittel vor dem Entfernen der Trümmer.

8. Verfahren nach Anspruch 7, wobei das Fällungsmittel ausgewählt ist aus der Gruppe bestehend aus einer Säure, einer Base, Methanol, 50%igem Methanol in Wasser und Kombinationen davon.

9. Verfahren nach einem der Ansprüche 1-8, wobei die Zellen Algenzellen in Form einer Algenzellsuspension, die zwischen 10 und 90 Gew.-% Wasser enthält, sind.

10. Verfahren nach einem der Ansprüche 2-9, wobei die Zellen Algenzellen in Form von ganzen, zerkleinerten oder zerstückelten Makroalgen sind.

11. Verfahren nach einem der vorhergehenden Ansprüche, wobei die ionische Flüssigkeit ausgewählt ist aus der Gruppe bestehend aus 1-Ethyl-3-methylimidazoliumchlorid, 1-Butyl-3-methylimidazoliumchlorid, 1-Ethyl-3-methylimidazoliumacetat, 1-Methyl-3-octylimidazoliumchlorid, 1-Hexyl-3-methylimidazoliumchlorid, 1-Hexyl-3-methylimidazoliumiodid, 1-Ethyl-3-methylimidazoliumtriflat und Kombinationen davon.

12. Verfahren nach einem der vorhergehenden Ansprüche, und ferner umfassend das Wiederholen des Zyklus der Schritte a) und b) zwischen 2 und 50 Mal, bevor mit Schritt c) fortgefahren wird.

13. Verfahren nach einem der vorhergehenden Ansprüche, und ferner umfassend das Entfernen von Wasser aus der ionische Flüssigkeit enthaltenden hydrophilen Phase des zweiten geklärten Zelllysats.

14. System zur Verarbeitung von Zellen, wobei das System umfasst:
eine Quelle von hydrophiler ionischer Flüssigkeit (11, 11a),
eine temperaturgesteuerte Lysereaktoreinheit (12) mit Mitteln zum Mischen (28);
eine temperaturgesteuerte Feststoffentfernungseinheit (13) mit einem Rührer (28);
eine Flüssigkeitstrennungseinheit (14); und
eine Wasserentfernungseinheit (16);
wobei:
die Lysereaktoreinheit (12) in Fließverbindung mit der Feststoffentfernungseinheit (13) steht, so dass ein rohes Zelllysat von der Lysereaktoreinheit (12) zur Feststoffentfernungseinheit (13) überführt werden kann und eine ionische Flüssigkeit (IL), die geklärtes Zelllysat enthält, von der Feststoffentfernungseinheit (13) zu der Lysereaktoreinheit (12) überführt werden kann;
wobei die Lysereaktoreinheit (12) mit einer Quelle von Zellen (10) in Verbindung steht und dafür konfiguriert ist, die Zellen und das IL-enthaltende geklärte Zelllysat zu mischen;
wobei die Feststoffentfernungseinheit (13) auf eine solche Weise in Fließverbindung mit der Flüssigkeitstrennungseinheit (14) steht, dass geklärtes Zelllysat entweder zu der Flüssigkeitstrennungseinheit (14) oder der Lysereaktoreinheit (12) überführt werden kann; wobei die Flüssigkeitstrennungseinheit (14) mit der Wasserentfernungseinheit (16) und einem Flüssigkeitsreservoir in Fließverbindung steht, so dass Flüssigkeit von der Flüssigkeitstrennungseinheit (14) zu der Wasserentfernungseinheit (16) oder dem Flüssigkeitsreservoir überführt werden kann;
wobei die Wasserentfernungseinheit (16) mit der Lysereaktoreinheit (12) in Fließverbindung steht, so dass die ionische Flüssigkeit in der Wasserentfernungseinheit (16) zu dem Lysereaktor (12) überführt werden kann; und
das System dafür konfiguriert ist, in Abhängigkeit von einem überwachten Wassergehalt des geklärten Lysats in der Feststoffentfernungseinheit (13) ein geklärtes Zelllysat zu der Flüssigkeitstrennungseinheit (14) zu überführen.

15. System nach Anspruch 14, wobei die Wasserentfernungseinheit (16) eine Destillationseinheit umfasst, die einen Sparger (25) umfasst, der zum Durchperlen einer Flüssigkeit in der Destillationseinheit mit einem erhitzten Gas konfiguriert ist.

16. System nach Anspruch 14, wobei die Destillationseinheit mit der Feststoffentfernungseinheit (13) in Fließverbindung steht und so konfiguriert ist, dass Dampf aus der Destillationseinheit zu einer Flüssigkeit kondensiert und zur Feststoffentfernungseinheit (13) überführt wird.

## Revendications

1. Procédé destiné au traitement de cellules, ledit procédé comprenant :
a) le mélange (100) de cellules avec un premier liquide ionique (IL) contenant un lysat cellulaire clarifié pour former un lysat cellulaire brut ;
b) le retrait des débris (200) dudit lysat cellulaire brut pour former un deuxième lysat cellulaire clarifié ;
c) la séparation des phases hydrophobe contenant des lipides et hydrophile contenant le liquide ionique (300) présentes dans le deuxième lysat cellulaire clarifié ; et
d) l'isolement de la phase hydrophobe contenant des lipides de la phase hydrophile contenant le liquide ionique ;
dans lequel le premier lysat cellulaire clarifié comprend au moins 65 % de liquide ionique hydrophile en poids et la quantité totale d'eau dans le lysat cellulaire brut formé à l'étape a) n'est pas supérieure à 45 % en poids.

2. Procédé selon la revendication 1, dans lequel les cellules sont des cellules d'algues.

3. Procédé selon la revendication 2, et comprenant en outre le mélange (302) d'un acide gras avec le deuxième lysat cellulaire clarifié avant de former des phases hydrophobe contenant des lipides et hydrophile contenant le liquide ionique, séparées.

4. Procédé selon la revendication 3, dans lequel ledit acide gras est ajouté en une quantité d'environ 0,5 % à environ 1,5 % du deuxième lysat cellulaire clarifié en poids.

5. Procédé selon les revendications 2 à 4, dans lequel lesdites cellules d'algues et ledit premier lysat cellulaire clarifié sont mélangés selon un rapport en poids compris entre 1:5 et 1:20.

6. Procédé selon l'une des revendications 1 à 5, dans lequel ledit mélange de cellules et de premier lysat cellulaire clarifié est effectué à une température comprise entre 20 °C et 50 °C pendant une durée comprise entre 1 et 60 minutes.

7. Procédé selon une quelconque revendication précédente, et comprenant en outre le mélange (201) du lysat cellulaire brut avec un agent de précipitation avant ledit retrait des débris.

8. Procédé selon la revendication 7, dans lequel ledit agent de précipitation est choisi dans le groupe constitué par un acide, une base, le méthanol, 50 % de méthanol dans l'eau, et leurs combinaisons.

9. Procédé selon l'une quelconque des revendications 1 à 8, dans lequel les cellules sont des cellules d'algues sous la forme d'une suspension de cellules d'algues comprenant entre 10 % et 90 % d'eau en poids.

10. Procédé selon l'une quelconque des revendications 2 à 9, dans lequel les cellules d'algues sont sous la forme de macroalgues entières, déchiquetées ou hachées.

11. Procédé selon une quelconque revendication précédente, dans lequel le liquide ionique est choisi dans le groupe constitué par le chlorure de 1-éthyl-3-méthylimidazolium, le chlorure de 1-butyl-3-méthylimidazolium, l'acétate de 1-éthyl-3-méthylimidazolium, le chlorure de 1-méthyl-3-octylimidazolium, le chlorure de 1-hexyl-3-méthylimidazolium, l'iodure de 1-hexyl-3-méthylimidazolium, le triflate de 1-éthyl-3-méthylimidazolium et leurs combinaisons.

12. Procédé selon une quelconque revendication précédente, et comprenant en outre la répétition du cycle des étapes a) et b) entre 2 et 50 fois avant de passer à l'étape c).

13. Procédé selon une quelconque revendication précédente, et comprenant en outre le retrait de l'eau de la phase hydrophile contenant le liquide ionique dudit deuxième lysat cellulaire clarifié.

14. Système destiné au traitement de cellules, ledit système comprenant :
une source de liquide ionique hydrophile (11, 11a),
une unité de réacteur de lyse à température contrôlée (12) comprenant des moyens destinés au mélange (28) ;
une unité de retrait de matières solides à température contrôlée (13) comprenant un agitateur (28) ;
une unité de séparation de liquide (14) ; et
une unité de retrait d'eau (16)
dans lequel :
ladite unité de réacteur de lyse (12) est en communication fluidique avec ladite unité de retrait de matières solides (13) de telle sorte qu'un lysat cellulaire brut peut être transféré de l'unité de réacteur de lyse (12) vers l'unité de retrait de matières solides (13) et qu'un liquide ionique (IL) contenant un lysat cellulaire clarifié peut être transféré de l'unité de retrait de matières solides (13) vers l'unité de réacteur de lyse (12) ;
ladite unité de réacteur de lyse (12) est en communication avec une source de cellules (10) et est configurée pour le mélange desdites cellules et dudit lysat cellulaire clarifié contenant l'IL ;
ladite unité de retrait de matières solides (13) est en communication fluidique avec ladite unité de séparation de liquide (14) de telle manière que le lysat cellulaire clarifié peut être transféré soit vers l'unité de séparation de liquide (14), soit vers l'unité de réacteur de lyse (12) ;
ladite unité de séparation de liquide (14) est en communication fluidique avec ladite unité de retrait d'eau (16) et un réservoir de fluide de telle sorte que du fluide peut être transféré de l'unité de séparation de liquide (14) vers l'unité de retrait d'eau (16) ou vers ledit réservoir de fluide ;
ladite unité de retrait d'eau (16) est en communication fluidique avec ladite unité de réacteur de lyse (12) de telle sorte que le liquide ionique dans l'unité de retrait d'eau (16) peut être transféré vers le réacteur de lyse (12) ; et
le système est configuré pour transférer un lysat cellulaire clarifié vers ladite unité de séparation de matières liquides (14) en fonction d'une teneur en eau suivie du lysat clarifié dans ladite unité de retrait de matières solides (13).

15. Système selon la revendication 14, dans lequel ladite unité de retrait d'eau (16) comprend une unité de distillation comprenant un dispositif de barbotage (25) configuré pour le barbotage d'un gaz chauffé dans un liquide dans l'unité de distillation.

16. Système selon la revendication 14, dans lequel ladite unité de distillation est en communication fluidique avec ladite unité de retrait de matières solides (13) et est configurée de telle sorte que la vapeur de l'unité de distillation est condensée en un liquide et transférée vers l'unité de retrait de matières solides (13).
